# FASCICULE DE BREVET EUROPEEN

(11) **EP 3 793 373 B1**
(45) Date de publication et mention de la délivrance du brevet: **06.11.2024**
(21) Numéro de dépôt: 19737159.4
(22) Date de dépôt: 14.05.2019
(51) Int. Cl.: A23L 33/14, A23K 10/18, A23K 50/75, A23K 50/10

(54) **PROBIOTIQUE POUR VOLAILLE**
PROBIOTIKUM FÜR GEFLÜGEL
PROBIOTIC FOR POULTRY

(30) Priorité: 15.05.2018 FR 1854046
(43) Date de publication de la demande: 24.03.2021
(60) Demande divisionnaire: 24185836.4
(73) Titulaire: Lesaffre et Compagnie, 75001 Paris (FR)
(72) Inventeur: AUCLAIR, Eric, 59710 AVELIN (FR); JULIEN, Christine, 31360 SEPX (FR); MARDEN, Jean-Philippe, 31670 Labège (FR)
(74) Mandataire: Plasseraud IP
(86) Numéro de dépôt international: PCT/FR2019/051097
(87) Numéro de publication internationale: WO 2019/220052

(56) Documents cités:
- CN-A- 104 988 089
- CN-A- 105 146 205
- FR-A1- 2 909 685
- US-A- 4 318 991
- US-A1- 2002 146 399
- K.-L. CHEN ET AL: "Effects of Bacillus subtilis var. natto and Saccharomyces cerevisiae mixed fermented feed on the enhanced growth performance of broilers", POULTRY SCIENCE, vol. 88, no. 2, 1 February 2009 (2009-02-01), Oxford, pages 309 - 315, XP055523492, ISSN: 0032-5791, DOI: 10.3382/ps.2008-00224
- W. CHEN ET AL: "Effects of Bacillus subtilis var. natto and Saccharomyces cerevisiae fermented liquid feed on growth performance, relative organ weight, intestinal microflora, and organ antioxidant status in Landes geese1", JOURNAL OF ANIMAL SCIENCE, vol. 91, no. 2, 1 February 2013 (2013-02-01), US, pages 978 - 985, XP055523490, ISSN: 0021-8812, DOI: 10.2527/jas.2012-5148
- A. W. ZHANG: "Effects of Graded Levels of Dietary Saccharomyces cerevisiae on Growth Performance and Meat Quality in Broiler Chickens", 10 January 2005 (2005-01-10), XP055523496, Retrieved from the Internet <URL:https://www.ajas.info/upload/pdf/18_110.pdf> [retrieved on 20181113]
- KANWAL RAFIQUE ET AL: "Effect of dietary supplementation of different levels of saccharomyces cerevisiae on growth performance and hematology in broiler", INDIAN JOURNAL OF ANIMAL RESEARCH, no. 00, 30 January 2018 (2018-01-30), INDIA, XP055523501, ISSN: 0367-6722, DOI: 10.18805/ijar.B-695
- HUANG: "Analyse quantitative de l'effet des levures vivantes sur le potentiel redox ruminal chez la vache laitière", 1 January 2016 (2016-01-01), XP055619593, Retrieved from the Internet <URL:http://www.journees3r.fr/IMG/pdf/Texte_14_affiche_Alimentation_Y-Huang.pdf> [retrieved on 20190906]
- JULIEN: "Addition de levures vivantes (ACTISAF Sc47) dans des rations à base de foin chez la vache laitière tarie : intérêts et limites.", 1 January 2011 (2011-01-01), XP055619594, Retrieved from the Internet <URL:http://www.journees3r.fr/IMG/pdf/Texte13_alim_Julien.pdf> [retrieved on 20190906]
- JEAN PHILIPPE MARDEN ET AL: "A Bioenergetic-Redox Approach to the Effect of Live Yeast on Ruminal pH during Induced Acidosis in Dairy Cow", AMERICAN JOURNAL OF ANALYTICAL CHEMISTRY, vol. 04, no. 10, 1 January 2013 (2013-01-01), pages 60 - 68, XP055619595, ISSN: 2156-8251, DOI: 10.4236/ajac.2013.410A1008
- ERIC PINLOCHE ET AL: "The Effects of a Probiotic Yeast on the Bacterial Diversity and Population Structure in the Rumen of Cattle", PLOS ONE, vol. 8, no. 7, 2 July 2013 (2013-07-02), pages e67824, XP055619596, DOI: 10.1371/journal.pone.0067824
- K&RCARON;?&ZCARON;OV? ET AL: "The effect of feeding live yeast cultures on ruminal pH and redox potential in dry cows as continuously measured by a new wireless device", CZECH J. ANIM. SCI, 1 January 2011 (2011-01-01), pages 37 - 45, XP055171511, Retrieved from the Internet <URL:http://www.agriculturejournals.cz/publicFiles/33676.pdf> [retrieved on 20150223]
- DANIEL SAUVANT ET AL: "Approche quantitative de l'acidose chez les ruminants", 1 January 2015 (2015-01-01), XP055619598, Retrieved from the Internet <URL:https://hal.archives-ouvertes.fr/hal-01413564/document> [retrieved on 20190906], DOI: 10.4267/2042/57937ï¿¿

## Description

### DOMAINE DE L'INVENTION

La présente divulgation concerne le domaine des probiotiques pour volaille.

L'invention concerne l'utilisation d'une levure active adaptée à un acide organique faible, chez la volaille.

Ladite levure est notamment utilisée pour améliorer les performances zootechniques de la volaille.

### ARRIÈRE-PLAN TECHNIQUE

Durant de nombreuses années, les antibiotiques qui, à des doses non médicamenteuses, ont un effet de facteur de croissance, ont été couramment utilisés pour améliorer les performances zootechniques dans l'élevage de volaille. Cependant, une telle utilisation en routine n'est pas sans conséquences, par exemple sur le développement des résistances aux antibiotiques de la part des bactéries. Aussi, en 2006, l'Union Européenne a-t-elle décidé d'interdire l'utilisation d'antibiotiques chez la volaille comme facteur de croissance, pour limiter l'apparition de phénomènes de résistance.

Les industriels ont donc cherché des alternatives aux antibiotiques. C'est ainsi que l'utilisation des probiotiques en volaille s'est étendue. La plupart des probiotiques commercialisés pour la volaille sont d'origine bactérienne principalement de la famille des *Bacillus,* compte tenu de leur capacité à sporuler et ainsi à résister aux contraintes thermiques élevées de la fabrication des aliments avicoles.

S'il a été constaté que les levures, notamment *Saccharomyces cerevisiae,* peuvent avoir un effet positif sur la croissance et les performances d'élevage des porcins et des bovins, leur impact sur la croissance et le gain de poids des volailles n'a pas été démontré et était souvent très limité et non significatif.

Par exemple, dans US2002/0146399, est proposé un produit pouvant être ajouté à l'alimentation animale notamment pour la volaille, comprenant de l'acide sorbique et au moins une culture de micro-organismes ayant une activité probiotique, telle que, par exemple, une culture de la levure *Saccharomyces cerevisiae.*

Dans WO97/00017 est proposé un probiotique, notamment utile pour des poules, contenant de la farine d'algues, de la levure sèche active et un composant minéral, la levure pouvant appartenir à l'espèce *Saccharomyces cerevisiae.*

En outre il n'existe actuellement dans l'Union Européenne aucune autorisation de levure comme probiotique pour les volailles.

Il existe donc un réel besoin de trouver des « probiotiques levures » efficaces permettant d'améliorer les performances de croissance, de gain de poids et l'indice de consommation (IC) de la volaille.

On entend par « probiotique levure », un probiotique comprenant une levure ou encore une levure qui a une fonction de probiotique. On peut également désigner le « probiotique levure » par « levure probiotique ».

### RESUME DE L'INVENTION

L'invention est telle que définie dans les revendications 1 à 7 ci-après.

Les Inventeurs ont développé et mis au point de nouvelles levures actives aux propriétés particulières, en ce sens qu'elles sont notamment adaptées à un acide organique faible.

Ainsi, la présente demande décrit des nouvelles levures actives adaptées à un acide organique faible.

La présente demande décrit également un probiotique pour volaille caractérisé en ce qu'il comprend une levure active adaptée à un acide organique faible.

Selon un aspect, la présente invention concerne l'utilisation d'une levure active adaptée à un acide organique faible telle que définie à la revendication 1 pour améliorer les performances zootechniques de la volaille.

On entend, au sens de la présente invention, par « amélioration des performances zootechniques de la volaille », une amélioration des performances de croissance, de gain de poids et l'indice de consommation (IC) de la volaille.

Selon un autre aspect, la présente invention concerne l'utilisation d'une levure active adaptée à un acide organique faible dans un aliment pour volaille.

De façon avantageuse, l'utilisation d'une levure adaptée selon l'invention permet d'éviter l'adjonction d'enzymes dans l'alimentation.

### DESCRIPTION DETAILLEE

La présente demande décrit une levure active *Saccharomyces cerevisiae* adaptée à un acide organique faible caractérisée en ce qu'elle est issue d'une souche choisie parmi les souches déposées à la CNCM sous les numéros 1-5128 et I-5131 le 31 août 2016, et l'activité selon le test A1 de la levure active non adaptée issue de la même souche choisie parmi I-5128 et I-5131, est supérieur ou égal à 1.

Le terme « levure » désigne des levures obtenues par culture d'une souche de levure.

La culture de la souche de levure est effectuée dans un milieu de culture approprié à la croissance de la souche de levure.

L'homme du métier est capable de déterminer quelle est la composition d'un milieu de culture approprié à une souche de levure donnée selon le protocole décrit dans le livre de référence « Yeast technology » par Gerald Reed et Tilak W. Nagodawithana (ISBN 0-442-31892-8) aux pages 284 à 293.

Un procédé de culture d'une souche et d'obtention d'une levure adaptée à un acide organique faible est décrit dans l'exemple 1.

Le terme « levure active », qui est synonyme de levure vivante, désigne une population de cellules de levure qui sont métaboliquement actives.

Par « levure active ... issue d'une souche », on entend une levure obtenue par culture et adaptation d'une souche.

Par « levure active adaptée à un acide organique faible », on entend une levure dont la tolérance à un acide organique faible donné est augmentée grâce à une précédente exposition à cet acide (Warth, 1988, Appl. Environ. Microbiol., 54 (8) : 2091-2095). La croissance et la capacité fermentaire de la levure adaptée ne sont ainsi pas inhibées lorsque la levure est en présence d'un acide organique faible pour au moins la seconde fois.

Au sens de la présente invention, les termes « levure active adaptée à un acide organique faible », « levure adaptée à un acide organique faible », « levure adaptée », « levure active adaptée » ont la même signification.

Par « acide organique faible », on entend l'acide organique en tant que tel et ses sels. Il est choisi parmi l'acide propionique et ses sels, notamment les sels de calcium ou de sodium. A titre d'exemples plus particuliers, on citera le propionate de calcium.

Un procédé d'obtention d'une levure adaptée à un acide organique faible est décrit dans l'exemple 1, basé sur le procédé décrit dans le brevet US 4 318 991.

L'adaptation des levures utilisées selon l'invention à la présence d'un acide organique faible est plus particulièrement effectuée lors des dernières heures de leur dernier stade de multiplication, par ajout de 0,1 g à 10 g d'acide organique faible et/ou leurs sels, par litre de milieu de culture.

La levure active *Saccharomyces cerevisiae* utilisée selon l'invention est plus particulièrement *Saccharomyces cerevisiae var. boulardii* également appelée *Saccharomyces boulardii.*

Il est décrit dans la présente demande une levure active adaptée, ou un probiotique la comprenant, dont le rapport de l'activité selon le test A1 (décrit ci-après) entre une levure dite « adaptée » et une levure non adaptée est supérieur ou égal à 1.

Ainsi, selon la présente invention, la levure active sélectionnée est une levure active, du genre *Saccharomyces,* qui se caractérise par un rapport de l'activité selon le test A1 entre cette levure active sélectionnée dite « adaptée » et la levure non adaptée, comme suit :

### Activité de la levure adaptée selon le test A1

Activité de la levure non adaptée selon le test A1 supérieur ou égal à 1.

Ledit test A1 correspond au protocole suivant :
on mesure pendant 2 heures le volume de CO₂ dégagé par la levure active avec le fermentomètre de Burrows et Harrisson suivant la méthode standard décrite aux pages 579 à 584 du livre de référence « Guide pratique d'analyses dans les industries de céréales » coordonné par B.Godon et W.Loisel (Ed Lavoisier - Tec & Doc - 1997 - ISBN. 2-7430-0123-2 - 2° édition) en utilisant le mode opératoire adapté suivant :
(a) Peser des lots de 20 g de farine, la farine étant une farine de froment ayant un temps de chute Hagberg de 200 à 300 secondes (tel que défini dans aux pages 680 et 681 du livre de référence cité ci-dessus), et les mettre dans des tubes propres et secs, puis ajouter dans chacun des tubes 2 g de saccharose et faire incuber ces tubes dans le râtelier du bain-marie, qui se trouve à 30°C, pendant au moins 30 minutes avant de commencer l'essai ;
(b) Prendre comme échantillon de levure l'équivalent de 1,066 g de matière sèche levure ;
(c) Préparer les échantillons délayés de levure dans 100 ml d'eau distillée (la méthode pour levure sèche est décrite à la page 583 dudit livre de référence) et ajouter 0,5 ml d'acide acétique à 6,6% (p/v) juste avant l'ajustement à 100 ml ;
(d) Pipeter les 15 ml de l'échantillon délayé bien mélangé nécessaires au malaxage, ces 15 ml apportant 1,066 g x 0,15 = 0,160 g de matières sèches levure ; et suivre pour le reste les instructions du mode opératoire décrit dans le livre de référence, en réalisant la mesure sur 2 heures.

Afin de tenir compte de l'effet du séchage sur l'activité de la levure, l'activité de la levure active selon le test A1 est définie comme suit :
- la levure testée est sous forme sèche, c'est-à-dire à une teneur en matières sèches d'au moins 90%, son activité correspond au volume de CO₂ mesuré.

La levure adaptée à l'acide organique faible utilisée selon l'invention, est obtenue par culture et adaptation d'une souche *Saccharomyces cerevisiae* choisie parmi les souches déposées auprès de la Collection Nationale de Cultures de Microorganismes, 25 rue du Docteur Roux 75724 Paris cedex 15, respectivement sous les numéros CNCM I-5128 et I-5131, le 31 août 2016.

Les Inventeurs ont en effet découvert de manière surprenante que des levures ayant subi un processus d'adaptation à un acide organique faible lors de leur culture sont plus efficaces chez la volaille que des levures n'ayant pas subi ce processus.

Ces levures dites « adaptées » permettent ainsi notamment d'améliorer les performances de croissance, de gain de poids, et l'indice de conversion de la consommation de l'aliment par rapport au gain de poids de la volaille.

On entend par « indice de conversion de la consommation de l'aliment par rapport au gain de poids » de la volaille, l'indice de consommation de la volaille. L'indice de consommation (IC) est la quantité d'aliment ingéré (en kilogrammes) par une volaille pour prendre 1 kilogramme de poids vif.

Selon un autre mode de réalisation particulier de l'invention, la levure adaptée à l'acide organique faible utilisée selon l'invention est sous forme sèche.

On entend par levure sèche, toute levure ayant un taux de matière sèche supérieur à 90%.

La levure sèche active est une levure vivante, séchée de façon à conserver son pouvoir fermentaire et lui assurer une conservation très longue. Ladite levure sèche active présente une teneur élevée en cellules vivantes de levure et peut se présenter sous différentes formes, par exemple sous forme de sphérules, de granules ou de poudre, toutes ces formes ayant une teneur moyenne en eau comprise entre 4 et 8%.

Le séchage est effectué selon toute méthode connue de l'homme du métier et compatible avec la viabilité des cellules, c'est-à-dire permettant de conserver une teneur élevée en cellules vivantes. L'homme du métier sait également que, selon le type de séchage effectué, la teneur en cellules vivantes est plus ou moins élevée, et les performances dans le test A1 peuvent être affectées.

Le poids de matière sèche est déterminé par les méthodes classiques bien connues de l'homme du métier.

Selon encore un autre mode de réalisation de l'invention, la levure active adaptée utilisée selon l'invention se présente sous forme de granulés, de microsphérules, de farine ou sous forme hydrodispersible.

La présente demande décrit un probiotique comprenant une levure active adaptée à un acide organique faible telle que définie ci-dessus.

Par « probiotique », on entend des micro-organismes vivants qui, lorsqu'ils sont ingérés en quantité suffisante, exercent des effets positifs sur la santé, au-delà des effets nutritionnels traditionnels.

Le probiotique tel que défini ci-dessus comprend au moins 90% en masse de la matière sèche de la levure active adaptée.

Le probiotique est plus particulièrement un probiotique pour volaille.

La présente demande décrit également un aliment pour volaille comprenant le probiotique décrit ci-dessus.

Le probiotique et l'aliment pour volaille peuvent également comprendre un ou plusieurs ingrédients autres que ladite levure active adaptée, comme notamment des vitamines, des minéraux alimentaires, des oligo-éléments, des enzymes alimentaires, des acidifiants, des extraits végétaux, des parois de levure et des graisses alimentaires, ainsi que, le cas échéant, d'autres micro-organismes à effet probiotique.

Selon les connaissances de l'homme du métier de l'élevage de volailles, la période de croissance d'un poulet est comprise de la naissance à 35 - 42 jours. Durant ce laps de temps, il passe d'une masse de 30 à 50 grammes à une masse de 2 à 3 kilogrammes, avec une masse qui est multipliée par 5 à 6 en 10 jours. Son alimentation, en termes de quantité, est fixée préférentiellement en fonction de son âge.

Selon un mode de réalisation particulier de l'invention et sur cette base de poids de la volaille, l'animal reçoit entre 10⁶ et 10¹¹ CFU de levure active adaptée à l'acide organique faible par kilogramme d'aliment par jour, de préférence entre 10⁹ et 10¹¹ CFU/kg d'aliment/jour, et encore plus de préférence entre 10⁹ et 10¹⁰ CFU/kg d'aliment/jour.

Le terme CFU ou UFC signifie unité formant colonie, un CFU ou UFC correspond à une colonie.

La présente demande décrit également une méthode pour améliorer les performances zootechniques de l'animal, notamment pour améliorer les performances de croissance, de gain de poids et l'indice de consommation de la volaille, consistant à fournir à l'animal une levure active adaptée à un acide organique faible, notamment entre 10⁶ et 10¹¹ CFU de levure active adaptée à un acide organique faible par kilogramme d'aliment et par jour, de préférence entre 10⁹ et 10¹¹ CFU/kg d'aliment/jour, et encore plus de préférence entre 10⁹ et 10¹⁰ CFU/kg d'aliment/jour.

Il a en effet été constaté que l'utilisation selon l'invention d'une levure adaptée chez la volaille permettait d'augmenter le gain de poids, la quantité d'aliment ingéré et d'améliorer l'indice de consommation de l'aliment par rapport au gain de poids, en particulier chez la volaille en croissance.

L'invention a également pour objet l'utilisation d'une levure active adaptée telle que définie ci-dessus, dans un aliment pour volaille.

Selon un autre mode de réalisation particulier de l'invention, la levure active adaptée à l'acide organique est mise en oeuvre sous une forme adaptée à une utilisation dans une matrice alimentaire, ladite matrice étant par exemple des granulés, de la farine, ou de l'eau de boisson.

La levure adaptée à l'acide organique faible est par exemple sous forme de granulés ou de farine lorsqu'elle est mélangée à l'aliment et sous forme hydrodispersible, lorsqu'elle est mélangée à l'eau de boisson.

Selon un autre aspect, l'invention concerne l'utilisation d'une levure active adaptée à un acide organique faible, dans laquelle la volaille pesant entre 30 et 3000 g, reçoit entre 10⁶ et 10¹¹ CFU de levure active adaptée à un acide organique faible par kilogramme d'aliment par jour, de préférence entre 10⁹ et 10¹¹ CFU/kg d'aliment/jour, et encore plus de préférence entre 10⁹ et 10¹⁰ CFU/kg d'aliment/jour.

L'utilisation de la levure adaptée suivant l'invention est notamment utile dans le domaine de l'élevage de volailles choisies dans le groupe comprenant :
- les oiseaux de basse-cour et gallinacées, et notamment les poules, y compris les poussins, les coqs et coquelets, les chapons et les poules pondeuses, les dindes et les dindons,
- les ansériformes et notamment les canards, les canes, les oies, et les faisans, les pintades, les cailles et les perdrix,
- les colombins, notamment les pigeons,
- les ratites, notamment les autruches, les émeus et les kiwis.

Le probiotique décrit dans la demande comprend au moins 90% en masse de matière sèche de la levure active sélectionnée dite « adaptée ».

La présente invention va être illustrée à l'aide des exemples de réalisation qui suivent, étant entendu que ceux-ci n'ont aucune portée limitative.

### EXEMPLES

### Exemple 1 : Procédé d'obtention d'une levure active sèche adaptée à un acide organique faible

### 1.1.Procédé de culture « standard »

Les levures peuvent être obtenues par une culture sur mélasse de betteraves ou de cannes par exemple, suivant le procédé discontinu alimenté (en anglais : fed-batch).

Le procédé de culture comporte une succession d'étapes de fermentation comme décrit aux pages 284 à 293 du livre de référence « Yeast technology » par Gerald Reed et Tilak W. Nagodawithana (ISBN 0-442-31892-8).

### 1.2.Procédé pour induire une adaptation

Le procédé pour induire l'adaptation à un acide organique faible correspond au procédé de culture standard ci-dessus, modifié comme il suit selon les étapes suivantes :
1. Acidification : 2 à 3 h avant la fin de la culture (cf. ci-dessus le procédé de culture « standard ») et avant la phase d'adaptation, ajuster le pH du milieu de culture à une valeur comprise entre 4,0 et 5,5 et le maintenir jusqu' à la fin de la phase d'adaptation, par exemple avec de l'acide sulfurique ;
2. Adaptation : ajouter dans le milieu de culture une quantité d'acide organique faible ou de son sel associé de façon à obtenir entre 1500 et 3000 ppm (partie par million) d'acide organique faible en poids dans le milieu de culture. La dose d'ajout d'acide faible est liée à la composition de l'acide faible ou de son sel associé utilisé. Le milieu de culture est dépourvu de tampon.

L'homme du métier saura ajuster le pH et la quantité d'acide organique faible à utiliser sans déployer des efforts excessifs.

### 1.3.Séchage

Les levures sèches actives sélectionnées, c'est-à-dire levures sèches présentant une teneur élevée en cellules vivantes de levure, sont obtenues par séchage selon une technique connue de l'homme du métier pour obtenir un taux de matière sèche supérieur à 90%.

### Exemple 2 : Mesure du rapport de l'activité selon le test A1 entre une levure adaptée et une levure non adaptée

Les levures a et b correspondent à 2 lots différents a et b de la souche de levure non adaptée déposée à la CNCM le 31 août 2016 sous le numéro I-5131, c'est-à-dire une levure n'ayant pas subi le procédé d'adaptation décrit dans l'exemple 1.2.

La levure dite « adaptée », selon le procédé décrit dans l'exemple 1.1 et 1.2, utilisée dans le test A1 correspond à 2 lots différents a et b de la même souche de levure I-5131.

Le test A1 est celui décrit *supra.*

### Résultats du test A1 :

**Tableau 1**

| Souches | Activité (ml de CO₂) |
|---|---|
| I-5131 non adaptée (a) | 103 |
| I-5131 non adaptée (b) | 113 |
| I-5131 adaptée (a) | 106 |
| I-5131 adaptée (b) | 132 |

### Rapport de l'activité selon les tests A1 entre la levure 1-5131 adaptée et la levure I-5131 non adaptée :

**Tableau 2**

| Lots | Rapport |
|---|---|
| Lot (a) | 1,029 |
| Lot (b) | 1,168 |

Conclusion : Les levures adaptées utilisées selon l'invention présentent ainsi un rapport supérieur à 1.

D'autres tests ont été effectués, tous lots confondus, sur respectivement :
- des lots de levures non adaptées issues de la souche I-5131,
- des lots de levures adaptées à l'acide propionique issues de la souche I-5131.

Les résultats obtenus sont donnés dans le tableau 3 ci-dessous :

| Souches | Activité (ml de CO₂) (moyenne) | Erreur Standard | Limite Inférieure | Limite Supérieure |
|---|---|---|---|---|
| I-5131 non adaptée | 109,571 | 2,173 | 105,140 | 114,003 |
| I-5131 adaptée à l'acide propionique | 121,667 | 1,659 | 118,282 | 125,051 |

Conclusion : les levures actives adaptées utilisées selon l'invention présentent un rapport supérieur à 1, tous lots confondus.

### Exemple 3 : Essais d'une levure dite adaptée, pour l'alimentation de la volaille

Les levures sèches actives adaptées présentent des propriétés avantageuses surprenantes dans le domaine de l'aviculture.

Ces propriétés avantageuses surprenantes sont illustrées par les résultats du test suivant sur des poussins.

Classiquement, selon les connaissances de l'homme du métier de l'élevage de volailles, l'alimentation utilisée pour les poussins est un blé viscosant, autrement dit un blé de qualité moyenne voire mauvaise. Ceci représente un challenge nutritionnel, autrement dit une sorte de stress. Selon les connaissances de l'homme du métier, l'un des moyens d'améliorer la digestibilité d'un tel blé est l'adjonction d'enzymes. Dans le cadre de la présente invention, il n'y a pas d'adjonction d'enzymes, ce qui représente un avantage non négligeable en termes de coût.

Dans ce test, les résultats obtenus par l'adjonction d'un probiotique selon l'invention à un aliment farine pour poussins sont comparés avec les résultats obtenus avec cet aliment sans additif, et avec un mélange de cet aliment avec la même levure sèche active non adaptée.

### Poussins

Le test a été effectué sur une période de 1 à 21 jours sur une population de poussins répondant aux critères suivants : poulets ROSS âgés au début de l'essai de 1 jour.

### Composition de l'aliment farine pour poussins

L'aliment farine consiste principalement en du blé et du maïs sans enzymes anti-viscosantes (comme expliqué ci-dessus) selon la composition suivante :

**Tableau 5**

| **Ingrédient** | **Quantité (%)** |
|---|---|
| Blé | 40,0 |
| Maïs | 39,8 |
| Farine de soja | 16,6 |
| DL méthionine | 0,1 |
| Di-phosphate de calcium | 1,5 |
| Carbonate de calcium | 1,0 |
| Prémélange vitamines - minéraux | 0,6 |
| Sel | 0,4 |

La composition des différents mélanges pour poussins est donnée dans le tableau 6 ci-dessous.

**Tableau 6**

| Mélange testé | Additif ajouté par g d'aliment farine |
|---|---|
| Témoin | / |
| Mélange levure non adaptée | 800 g/T soit 10⁷ CFU/g d'aliment de la levure sèche active I-5128 |
| Mélange levure adaptée | 800 g/T soit 10⁷ CFU/g d'aliment de la levure sèche active adaptée I-5128 |

### Protocole du test

La population de poussins a été divisée de manière aléatoire en 2 réplicats de 3 lots de 6 cages contenant chacune 20 poussins, chaque lot de poussins recevant un mélange à tester selon le tableau 5.

A partir du premier jour, les poussins de chaque groupe ont été alimentés *ad libitum* avec le mélange correspondant au lot d'essai. Les poussins disposaient également *ad libitum* d'eau. Aucune autre source d'alimentation n'était mise à la disposition des poussins.

La quantité de farine consommée par chaque groupe était mesurée.

A différents moments durant le test, le poids vivant des poussins était mesuré, respectivement à J0 (poulet de 1 jour), J+7 j ours, J+ 14 jours J+21 jours.

### Résultats :

Les résultats du test, à J+21 jours, sont donnés dans le tableau 7, dans lequel :
- FI-x signifie la quantité totale moyenne (en g) de mélange consommé par poussin du groupe donné après x j ours, en l'occurrence ici FI-21, après 21 jours ;
- LW-x signifie le poids vivant moyen (en g) des poussins du groupe donné après x jours, en l'occurrence ici LW-21 après 21 jours.
- FCR-x signifie le rapport entre, d'une part, le gain de poids vivant moyen (en g) des poussins du groupe donné après x jours et, d'autre part, la quantité totale moyenne de mélange consommé par poussin dudit groupe après x jours, en l'occurrence ici FCR-21 après 21 jours.

| | **Control** | **I-5128** | **I-5128 adaptée** | **SEM** | **ANOVA (P)** |
|---|---|---|---|---|---|
| Initial LW | 40 | 40 | 40 | | |
| LW-21 | 516,2a | 514,6a | 545,2b | 6,9 | **0,036** |
| FI-21 | 820,9 | 814,3 | 833,6 | 7,8 | 0,551 |
| FCR-21 | 1,67a | 1,65a | 1,60b | 0,01 | **0,021** |

SEM signifie standard error of the mean, appellation anglo-saxonne de l'écart type de la moyenne.

### ANOVA signifie ANalysis Of VAriance

Les différentes lettres utilisées a, b indiquent si les valeurs sont statistiquement différentes ou non. Ainsi les résultats ne sont pas significativement différents lorsque la même lettre est reportée (p > 0,05).

Conclusion : Les résultats montrent que la supplémentation avec une levure adaptée améliore significativement les résultats de croissance (LW21) et de conversion alimentaire (FCR21) des poulets alors que la levure non adaptée n'a pas d'effet.

## Revendications

1. Utilisation d'une levure active *Saccharomyces cerevisiae* adaptée à un acide organique faible, ladite levure adaptée présentant les caractéristiques suivantes :
- elle est issue d'une souche choisie parmi les souches déposées à la CNCM respectivement sous les numéros I-5128 et I-5131 le 31 août 2016,
- le rapport entre l'activité selon le test A1 de ladite levure active adaptée issue d'une souche choisie parmi I-5128 et I-5131, et l'activité selon le test A1 de la levure active non adaptée issue de la même souche choisie parmi I-5128 et I-5131, est supérieur ou égal à 1, ledit test A1 correspondant au protocole tel que décrit dans la description en page 4, lignes 6-30,
- elle est obtenue selon le procédé décrit dans l'exemple 1 de la description,
- elle présente une tolérance à un acide organique faible augmentée grâce à une précédente exposition à cet acide de sorte que la croissance et la capacité fermentaire de ladite levure adaptée ne sont pas inhibées lorsque la levure est en présence dudit acide organique faible pour au moins la seconde fois, ledit acide organique faible étant choisi parmi l'acide propionique et ses sels,
pour améliorer les performances de croissance, de gain de poids et l'indice de consommation (IC) de la volaille.

2. Utilisation selon la revendication 1, **caractérisée en ce que** la levure est adaptée aux sels de calcium ou de sodium de l'acide propionique.

3. Utilisation selon la revendication 2, **caractérisée en ce que** la levure est adaptée au propionate de calcium.

4. Utilisation selon l'une quelconque des revendications 1 à 3, **caractérisée en ce que** la levure active adaptée est sous forme sèche.

5. Utilisation selon l'une quelconque des revendications 1 à 4, **caractérisée en ce qu'**elle se présente sous forme de granulés, de microsphérules, de farine ou sous forme hydrodispersible.

6. Utilisation selon l'une quelconque des revendications 1 à 5, dans laquelle la levure est dans un aliment pour volaille.

7. Utilisation selon la revendication 6, dans laquelle la volaille reçoit entre 10⁶ et 10¹¹ CFU de levure active adaptée par kilogramme d'aliment par jour, de préférence entre 10⁹ et 10¹¹ CFU/kg d'aliment/jour, et encore plus de préférence entre 10⁹ et 10¹⁰ CFU/kg d'aliment/j our.

## Patentansprüche

1. Verwendung einer aktiven Hefe Saccharomyces cerevisiae, die an eine schwache organische Säure angepasst ist, wobei die angepasste Hefe die folgenden Merkmale aufweist:
- sie stammt von einem Stamm, der aus den Stämmen gewählt ist, die am 31. August 2016 unter den Nummern I-5128 bzw. I-5131 bei der CNCM hinterlegt wurden,
- das Verhältnis zwischen der Aktivität gemäß Test A1 der angepassten aktiven Hefe, die von einem unter I-5128 und I-5131 gewählten Stamm stammt, und der Aktivität gemäß Test A1 der nicht angepassten aktiven Hefe, die von demselben unter I-5128 und I-5131 gewählten Stamm stammt, ist größer oder gleich 1, wobei der Test A1 dem Protokoll entspricht, wie es in der Beschreibung auf Seite 4, Zeilen 6-30, beschrieben ist,
- sie wird nach dem in Beispiel 1 der Beschreibung beschriebenen Verfahren erhalten,
- sie weist eine erhöhte Toleranz gegenüber einer schwachen organischen Säure aufgrund einer vorherigen Exposition gegenüber dieser Säure auf, so dass das Wachstum und die Fermentationsfähigkeit der angepassten Hefe nicht gehemmt werden, wenn sich die Hefe wenigstens zum zweiten Mal in Gegenwart der schwachen organischen Säure befindet, wobei die schwache organische Säure aus Propionsäure und ihren Salzen gewählt ist,
zur Verbesserung der Wachstumsleistung, der Gewichtszunahme und des Futterverwertungsindex (IC) des Geflügels.

2. Verwendung nach Anspruch 1, **dadurch gekennzeichnet, dass** die Hefe an die Calcium- oder Natriumsalze der Propionsäure angepasst ist.

3. Verwendung nach Anspruch 2, **dadurch gekennzeichnet, dass** die Hefe an Calciumpropionat angepasst ist.

4. Verwendung nach einem der Ansprüche 1 bis 3, **dadurch gekennzeichnet, dass** die angepasste aktive Hefe in Trockenform vorliegt.

5. Verwendung nach einem der Ansprüche 1 bis 4, **dadurch gekennzeichnet, dass** diese in Form von Granulat, Mikrokugeln, Mehl oder in wasserdispergierbarer Form vorliegt.

6. Verwendung nach einem der Ansprüche 1 bis 5, wobei sich die Hefe in einem Geflügelfutter befindet.

7. Verwendung nach Anspruch 6, wobei das Geflügel zwischen 10⁶ und 10¹¹ CFU (KbE) angepasste aktive Hefe pro Kilogramm Nahrung pro Tag erhält, bevorzugt zwischen 10⁹ und 10¹¹ CFU/kg Nahrung/Tag, und noch stärker bevorzugt zwischen 10⁹ und 10¹⁰ CFU/kg Nahrung/Tag.

## Claims

1. The use of a *Saccharomyces cerevisiae* active yeast adapted to a weak organic acid, said adapted yeast having the following features:
- it is derived from a strain chosen from the strains deposited at the CNCM, respectively under numbers I-5128 and I-5131 on August 31, 2016,
- the ratio between the activity according to the A1 test of said adapted active yeast derived from a strain chosen from I-5128 and 1-5131, and the activity according to the A1 test of the non-adapted active yeast derived from the same strain chosen from I-5128 and I-5131 is greater than or equal to 1, said A1 test corresponding to the protocol as described in the specification page 4, line 6-30,
- it is obtained according to the method described in example 1 of the specification,
- it presents an increased tolerance to a weak organic acid by virtue of a previous exposure to this acid such that the growth and the fermentative capacity of said adapted yeast are not inhibited when the yeast is in the presence of said weak organic acid for at least the second time, said weak organic acid being chosen from propionic acid and salts thereof,
for improving the growth and weight gain performances and the consumption index (CI) of poultry.

2. The use as claimed in claim 1, **characterized in that** the yeast is adapted to the calcium or sodium salts of propionic acid.

3. The use as claimed in claim 2, **characterized in that** the yeast is adapted to the calcium propionate.

4. The use as claimed in any one of claims 1 to 3, **characterized in that** the adapted active yeast is in dry form.

5. The use as claimed in any one of claims 1 to 4, **characterized in that** it is in the form of granules, microspherules, meal or in water-dispersible form.

6. The use as claimed in any one of claims 1 to 5, wherein the yeast is in a feed for poultry.

7. The use as claimed in claim 6, wherein the poultry bird receives between 10⁶ and 10¹¹ CFU of adapted active yeast per kilogram of feed per day, preferably between 10⁹ and 10¹¹ CFU/kg of feed/day, and even more preferably between 10⁹ and 10¹⁰ CFU/kg of feed/day.
